(19) Europäisches Patentamt
European Patent Office
Office européen des brevets

(11) **EP 3 851 092 A1**

(12) **EUROPEAN PATENT APPLICATION**
published in accordance with Art. 153(4) EPC

(43) Date of publication:
**21.07.2021 Bulletin 2021/29**

(21) Application number: **19861039.6**

(22) Date of filing: **12.09.2019**

(51) Int Cl.:
*A61K 8/25* *(2006.01)*  *A61K 8/02* *(2006.01)*
*A61K 8/73* *(2006.01)*  *A61Q 1/02* *(2006.01)*
*A61Q 1/12* *(2006.01)*

(86) International application number:
**PCT/JP2019/035935**

(87) International publication number:
**WO 2020/054810 (19.03.2020 Gazette 2020/12)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME
KH MA MD TN**

(30) Priority: **12.09.2018   JP 2018170746
15.04.2019   JP 2019076979**

(71) Applicant: **Nissan Chemical Corporation
Tokyo 103-6119 (JP)**

(72) Inventors:
• **IMOTO Takayuki**
  **Funabashi-shi, Chiba 274-0052 (JP)**
• **FUJITA Shun**
  **Funabashi-shi, Chiba 274-8507 (JP)**
• **KATSUYA Mutsuhiro**
  **Funabashi-shi, Chiba 274-0052 (JP)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(54) **FUNCTIONAL COMPLEX POLYSACCHARIDE PARTICLE**

(57)    An object is to provide a natural material-derived particle which is a natural material and soft, and is excellent in light scattering properties.

This is to provide a particle having an uneven structure on a surface thereof, and containing cellulose or a cellulose derivative and a clay mineral as main components, and a method for producing the same. Such a particle is soft and excellent in light scattering properties, and in particular, is excellent in a defocusing effect when it is formulated in cosmetics such as foundations, so that it is useful as cosmetics.

EP 3 851 092 A1

**Description**

TECHNICAL FIELD

The present invention relates to a particle which comprises having an uneven structure on a surface thereof, and containing cellulose or a cellulose derivative as a main component, a method for producing the same, and cosmetics containing said particles.

BACKGROUND ART

Porous fine particles have been used in a wide range of fields as various kinds of additives such as abrasives, matting agents (light diffusing agents), carriers, water absorbing materials, fillers, anti-blocking agents, etc., depending on the material, shape, physical properties, etc.

Among porous fine particles, microbeads generally refer to fine particles (particles having several $\mu$m to several hundred $\mu$m) made of plastics such as polyethylene, polypropylene, etc., and have widely been used for fragrances and cosmetics such as toiletry products (for example, face wash, body wash), oral care products (for example, dentifrices), cosmetics (for example, foundation), etc. Specifically, microbeads have been used as a scrubbing agent to enhance a massage effect and a cleaning effect in toiletry products such as face wash, body wash, etc., and in cosmetics such as foundation, etc., for example, they are used for developing a defocus effect (also referred to as a soft focus effect) that scatters reflected light in multiple directions, and corrects defects of skins such as spots, wrinkles, freckles, color unevenness, and the like (for example, see Patent Documents 1 and 2).

However, in recent years, it has been feared that microbeads pass through sewage treatment and flow out into rivers and oceans, and are taken up by living organisms to adversely affect the ecosystem. Since it is almost impossible to collect the microbeads once they flow into the environment, it has been required to replace the microbeads made of plastics with an environment-friendly material such as biodegradable plastic, a natural material, etc. As such a material, for example, porous resin fine particles containing a polyester-based thermoplastic resin having biodegradability as a main component have been reported (for example, see Patent Document 3). Also, as microbeads derived from natural materials, cellulose particles have been known (for example, see Patent Document 4).

PRIOR ART DOCUMENT

PATENT DOCUMENTS

> Patent Document 1: JP 2016-40264A
> Patent Document 2: WO 2010/092890
> Patent Document 3: WO 2017/056908
> Patent Document 4: JP Hei.9-132601A

SUMMARY OF THE INVENTION

PROBLEMS TO BE SOLVED BY THE INVENTION

However, since existing cellulose particles are hard and inferior in light scattering property, further improvement of these properties has been demanded for use in fragrances and cosmetics. That is, an object of the present invention is to provide a natural material-derived particle which is a natural material and is soft, and has excellent light scattering properties.

MEANS TO SOLVE THE PROBLEMS

As a result of intensive studies by the present inventors, they have found that particles obtained by spray-drying a dispersion of cellulose or a cellulose derivative and a clay mineral are soft since they have an uneven structure on the surface thereof, excellent in light scattering properties, and, in particular, excellent in a defocus effect at the time of being formulated in cosmetics such as foundations, whereby the present invention has been completed. The present invention is as follows.

(1) A particle which comprises having an uneven structure on a surface thereof, and containing cellulose or a cellulose derivative and a clay mineral as main components.

(2) The particle described in the above-mentioned (1), which has a wrinkle-like or fold-like uneven structure on a surface thereof.

(3) The particle described in the above-mentioned (1) or (2), wherein a void ratio is in a range of 5 to 50%.

(4) The particle described in any of the above-mentioned (1) to (3), wherein a particle size is in a range of 0.5 to 500 μm.

(5) The particle described in any of the above-mentioned (1) to (4), wherein a hardness is in a range of 0.1 to 5 MPa.

(6) The particle described in any of the above-mentioned (1) to (5), wherein a light scattering rate is in a range of 50 to 230%.

(7) The particle described in any of the above-mentioned (1) to (6), wherein, in a measurement of a distribution of reflected light, a ratio of an intensity of the reflected light in a vicinity of a specular reflection to incident light with respect to an intensity of the reflected light in a vicinity of the incident light is in a range of 0.5 : 1 to 2 : 1.

(8) The particle described in any of the above-mentioned (1) to (7), wherein the particle contains 0.1 to 20 parts by mass of the clay mineral with respect to 1 part by mass of the cellulose or cellulose derivative.

(9) The particle described in any of the above-mentioned (1) to (8), wherein the cellulose is a crystalline cellulose.

(10) The particle described in any of the above-mentioned (1) to (9), wherein the clay mineral is at least one selected from the group consisting of talc, kaolin and sericite.

(11) A method for producing a particle having an uneven structure on a surface thereof, and containing cellulose or a cellulose derivative and a clay mineral as main components, which comprises a step of obtaining a dispersion of cellulose or a cellulose derivative and a clay mineral, and a step of spray-drying the obtained dispersion.

(12) The producing method described in the above-mentioned (11), wherein the dispersion is obtained by physical pulverization of the cellulose or the cellulose derivative and the clay mineral.

(13) The producing method described in the above-mentioned (11) or (12), wherein a concentration of a solid content including the cellulose or the cellulose derivative and the clay mineral in the dispersion is 0.5 to 20% by mass.

(14) The producing method described in any of the above-mentioned (11) to (13), wherein the dispersion contains 0.1 to 20 parts by mass of the clay mineral with respect to 1 part by mass of the cellulose or the cellulose derivative.

(15) A cosmetic which comprises the particle described in any of the above-mentioned (1) to (10), or the particle obtained by the producing method described in any of the above-mentioned (11) to (14).

EFFECTS OF THE INVENTION

The particle of the present invention has an uneven structure on the surface thereof (that is, since pores or voids are appropriately present), so that it is soft, and is suitable for adding to cosmetics that comes into direct contact with skin. The particle of the present invention has also excellent optical characteristics (light scattering properties) that the incident light is uniformly light scattered, so that it can be expected to exhibit a defocus effect (also called a soft focus effect) by formulating it into cosmetics such as foundations, etc.

BRIEF DESCRIPTION OF THE DRAWINGS

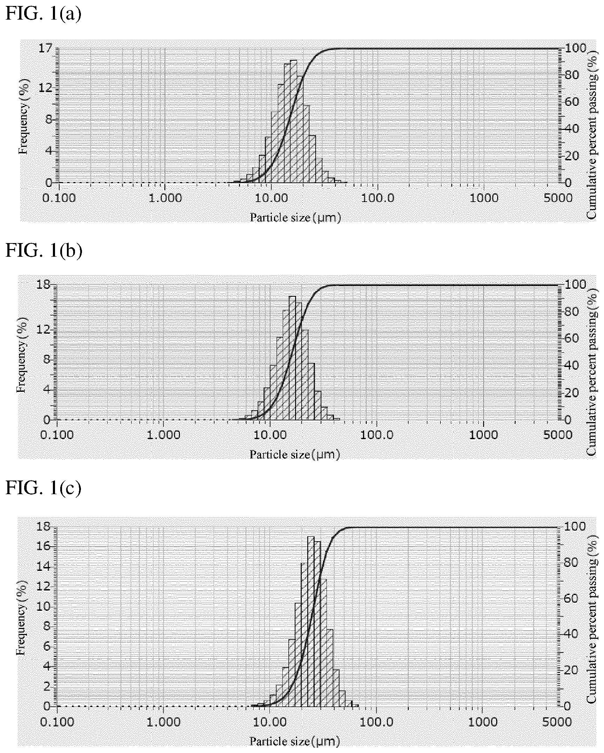

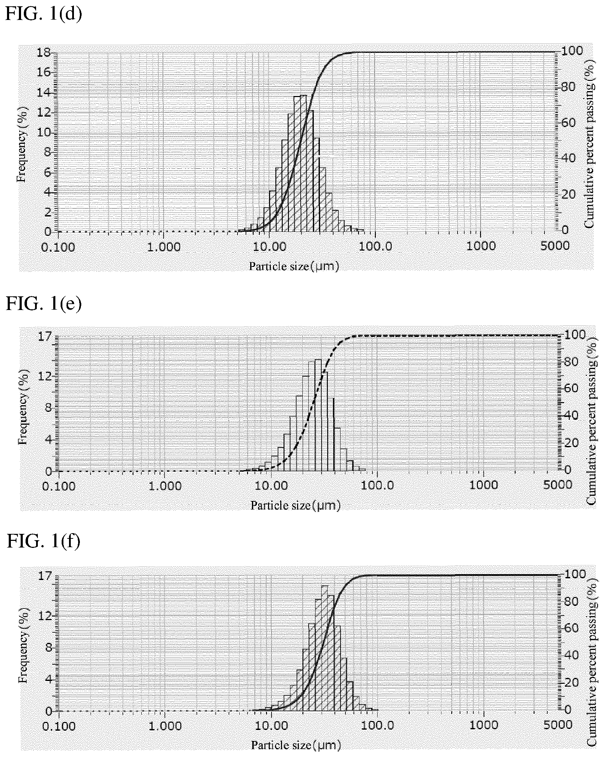

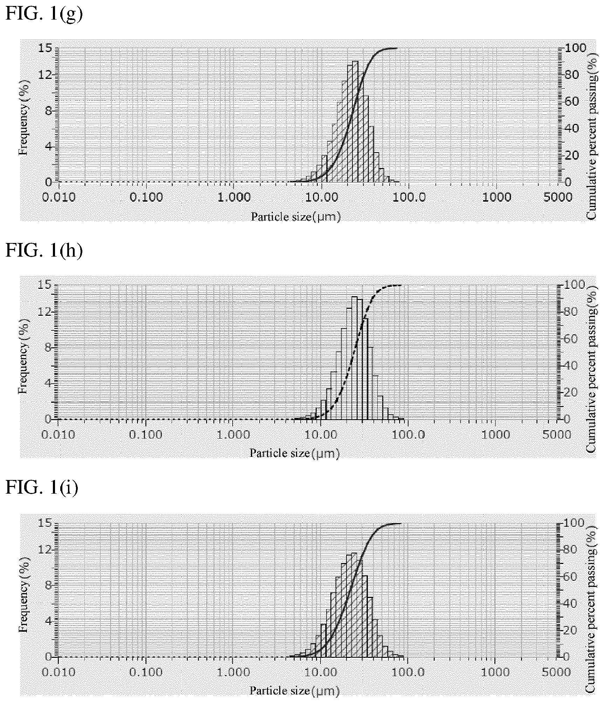

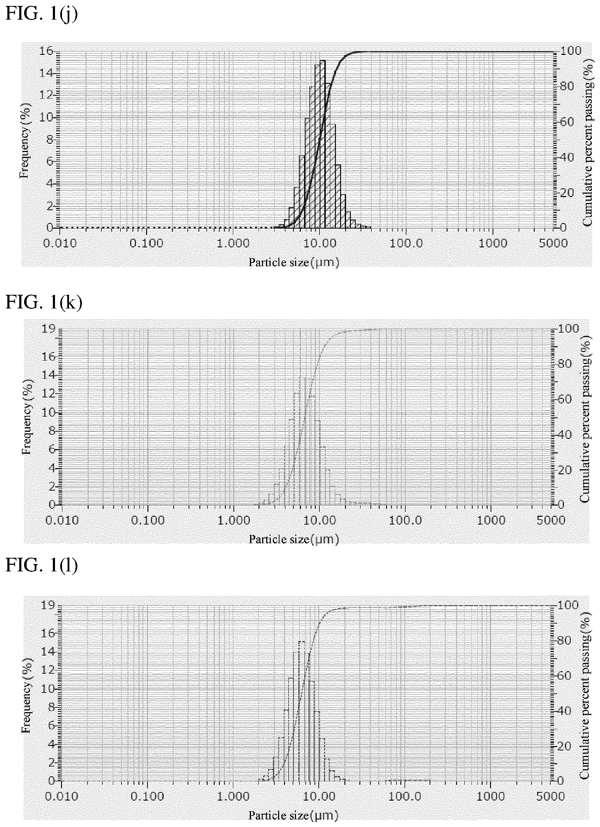

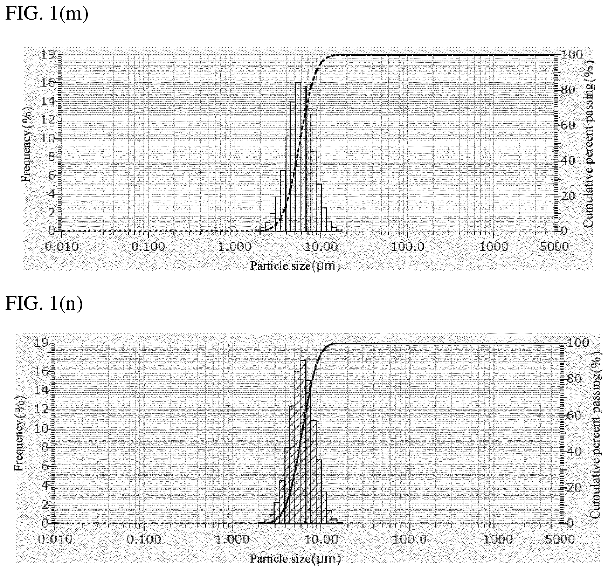

FIGs. 1(a) to (n) are volume particle size distributions and cumulative percent passing of particles obtained in Examples 1 to 14 in order.

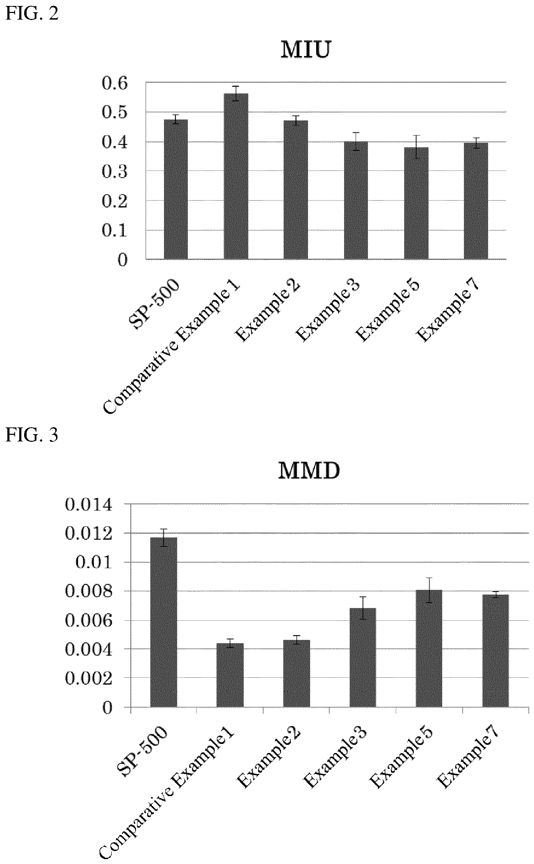

FIG. 2 is a graph showing the results of evaluating particles obtained in Examples 2, 3, 5 and 7, and commercially available Nylon particles as a control, by an average frictional coefficient (MIU) obtained by a friction tester.

FIG. 3 is a graph showing the results of evaluating particles obtained in Examples 2, 3, 5 and 7, and commercially available Nylon particles as a control, by deviation of an average frictional coefficient (MMD) obtained by a friction tester.

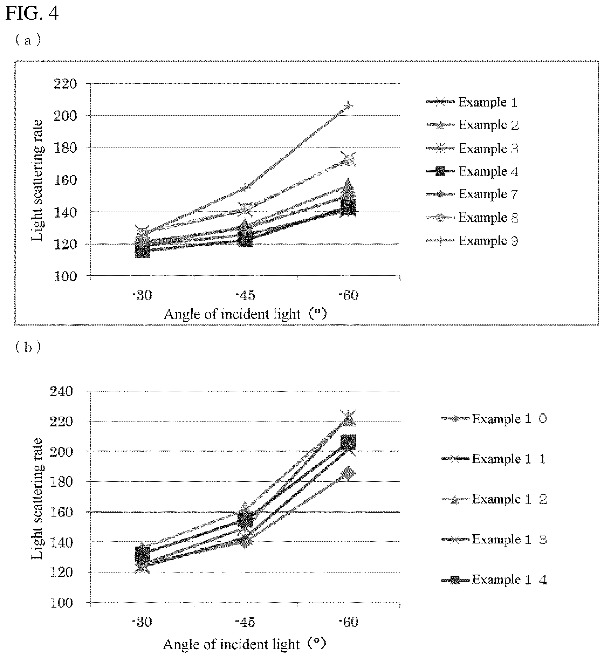

FIG. 4(a) is a graph showing a correlation between an angle of an incident light and a light scattering rate in a measurement of a distribution of reflected light of particles obtained in Examples 1 to 4 and Examples 7 to 9, and (b) is a graph showing a correlation between an angle of an incident light and a light scattering rate in a measurement of a distribution of reflected light of particles obtained in Examples 10 to 14.

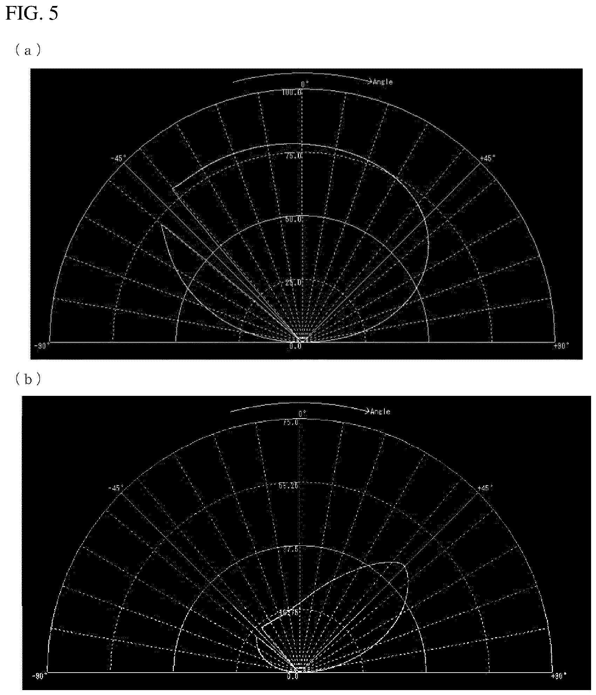

FIG. 5(a) is a drawing of a distribution of reflected light of the particles obtained in Example 2 at an angle of an incident light of -45°, and (b) is a drawing of a distribution of reflected light of CELLULOBEADS D-10 (Daito Kasei Kogyo Co., Ltd.) as Comparative Example at an angle of an incident light of -45°.

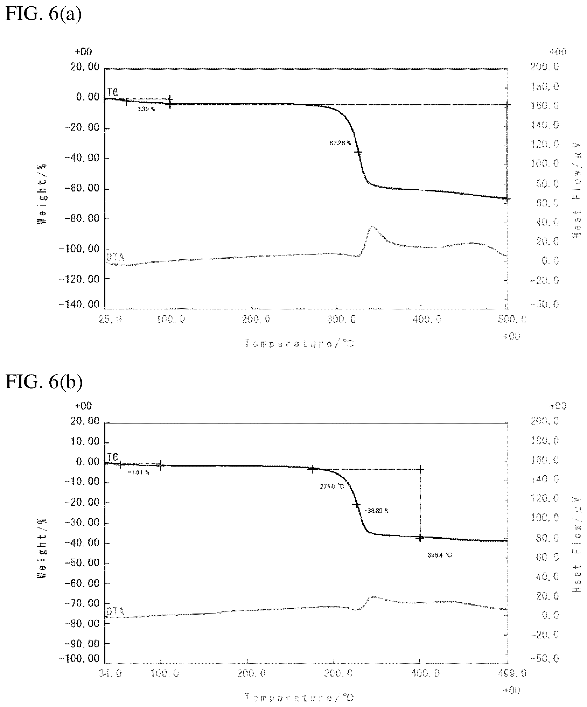

FIG. 6(a) is a graph showing change in weight and change in calorific value in thermogravimetric measurement-differential thermal analysis (TG-DTA) of particles obtained in Example 1, (b) is a graph showing change in weight and change in calorific value in TG-DTA of particles obtained in Example 2, (c) is a graph showing change in weight and change in calorific value in TG-DTA of particles obtained in Example 11, (d) is a graph showing change in weight and change in calorific value in TG-DTA of particles obtained in Example 12, (e) is a graph showing change in weight and change in calorific value in TG-DTA of particles obtained in Example 13, and (f) is a graph showing change in weight and change in calorific value in TG-DTA of particles obtained in Example 14.

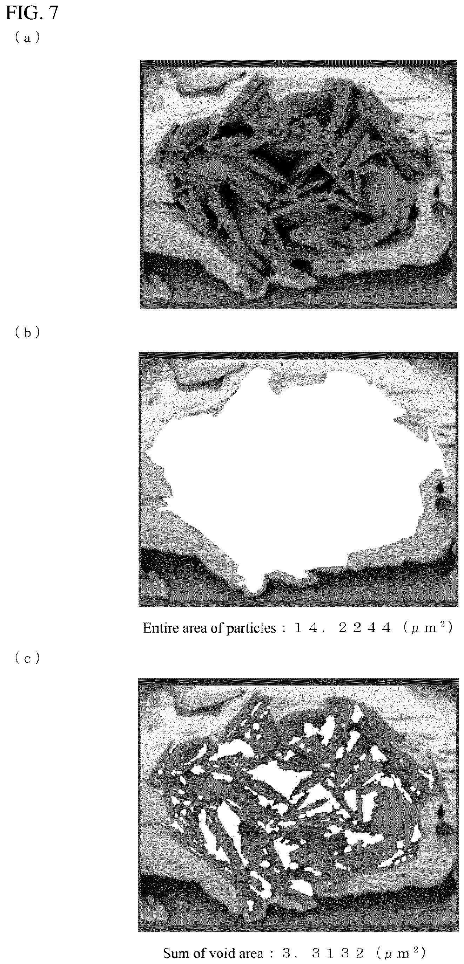

FIG. 7(a) is an SEM photograph of a cross section of particles obtained in Example 12, (b) is an SEM photograph highlighting entire cross section (cross-sectional area) of particles used for calculating the void ratio thereof, and (c) is an SEM photograph highlighting the void portion of the cross section of the particles used for calculating the void ratio.

EMBODIMENTS TO CARRY OUT THE INVENTION

<Particle>

The present invention relates to a particle which comprises having an uneven structure on a surface thereof, and containing cellulose or a cellulose derivative and a clay mineral as main components. The present invention preferably relates to a particle which comprises having a wrinkle-like or fold-like uneven structure on a surface thereof, and containing cellulose or a cellulose derivative and a clay mineral as main components. "Having a wrinkle-like or fold-like uneven structure" means that when an enlarged image of a particle is observed, the surface thereof is not smooth and has groove-shaped lines which have a wrinkle-like or fold-like appearance.

The particles of the present invention contain cellulose or a cellulose derivative as a main component. The cellulose or the cellulose derivative used in the present invention may be mentioned natural fibers such as wool, cotton, silk, hemp, pulp, etc., those derived from regenerated fibers such as rayon, polynosic, cupra (Bemberg (Registered Trademark)), lyocell (Tencel (Registered Trademark)), etc., or cellulose produced by bacteria. In addition, it may be derived from cellulose composite fibers of cellulose fibers and synthetic fibers (for example, polyolefin-based fibers such as polyethylene, polypropylene, etc.).

As the cellulose or the cellulose derivative to be used in the present invention, there may be mentioned those derived from natural fibers, for example, those derived from plants such as wood, bamboo, hemp, jute, kenaf, cotton, beet, agricultural waste products, etc., in particular, there may be mentioned those derived from broad-leaved tree, acicular tree or bamboo. Also, it is preferable to use a material in which $\alpha$-cellulose obtained from such a fibrous plant may be partially depolymerized with an acid and purified, for example, crystalline cellulose.

Also, in the present invention, it is preferable to use cellulose nanofibers as the cellulose or the cellulose derivative. The "cellulose nanofiber (CNF)" is a fiber obtained by defibrating cellulose fibers to a nanosize level, and is generally a fiber having a fiber width of about 4 to 200 nm and a fiber length of about 5 $\mu$m or more. Such cellulose nanofibers can be prepared by a known method and can be obtained as a commercially available product. For example, it can be obtained from suppliers such as Daio Paper Corporation and Chuetsu Pulp & Paper Co., Ltd., etc.

The particles of the present invention also contain a clay mineral as a main component. The clay mineral used in the present invention means a natural or synthetic layered silicate mineral, and is not particularly limited as long as it swells in water and is ion-exchangeable. Examples thereof may be mentioned talc, kaolin, sericite, montmorillonite, kaolinite, mica such as muscovite, phlogopite, synthetic mica, lepidolite and biotite, vermiculite, zeolite, bentonite, smectite, chlorite, illite, glauconite, and the like. Among these, from the viewpoint of combination with the cellulose or the cellulose derivative and application of the particles to cosmetics, at least one selected from the group consisting of talk, kaolin and sericite is preferable. These clay minerals can be obtained from suppliers as an additive for pharmaceuticals or cosmetics.

The above-mentioned layered silicate mineral is contained as a main component of the clay mineral, and a content thereof is generally 60% or more, preferably 75% or more, and most preferably 80% or more.

In the present invention, "containing cellulose or a cellulose derivative and a clay mineral as main components" means that a ratio (mass standard) of the cellulose or the cellulose derivative and the clay mineral occupied in the particles exceeds 50% by mass. The ratio (mass standard) of the cellulose or the cellulose derivative and the clay mineral is preferably 60% by mass or more, more preferably 70% by mass or more, further preferably 80% by mass or more, and particularly preferably 90% by mass or more. In the most preferred embodiment, the particles of the present invention comprise the cellulose or the cellulose derivative and the clay mineral alone.

In the present invention, a formulation ratio of the cellulose or the cellulose derivative and the clay mineral is not particularly limited as long as it can accomplishes the effect of the present invention, and typically contains 0.1 to 20 parts by mass of the clay mineral, preferably 0.2 to 20 parts by mass, more preferably 0.5 to 15 parts by mass, and particularly preferably 1 to 10 parts by mass with respect to 1 part by mass of the cellulose or the cellulose derivative.

As the component(s) other than the cellulose or the cellulose derivative and the clay mineral contained in the particles, there may be mentioned, for example, magnesium carbonate, calcium carbonate, aluminum silicate, barium silicate, calcium silicate, magnesium silicate, strontium silicate, tungstate metal salt, barium sulfate, calcined calcium sulfate, calcium phosphate, fluoroapatite, hydroxyapatite, ceramic powder, metallic soap (for example, zinc myristate, calcium palmitate, aluminum stearate), rouge (Bengala), yellow iron oxide, black iron oxide, ultramarine, Prussian blue, carbon black, titanium oxide, fine particle and ultrafine particle titanium oxide, zinc oxide, fine particle and ultrafine particle zinc oxide, alumina, silica, fumed silica (ultrafine particle silicic anhydride), titanated mica, fish scale, boron nitride, photochromic pigments, synthetic fluorophlogopite, fine particle complex powders, inorganic powders with various kinds of sizes and shapes such as gold, aluminum, etc., and a powder, etc. in which these are hydrophobized or hydrophilized by treatment(s) with various kinds of surface treatment agent such as silicone including hydrogen silicone, cyclic hydrogen silicone, etc., or other silanes or a titanium coupling agent, and the like.

A particle size of the particles of the present invention can be appropriately set depending on the desired use of the particles and, for example, it is distributed in the range of 0.5 to 500 μm, preferably in the range of 1 to 200 μm, more preferably in the range of 2 to 100 μm, and particularly preferably in the range of 5 to 80 μm, and an average particle size is, for example, in the range of 5 to 40 μm, and preferably in the range of 5 to 30 μm. Here, in the present invention, the particle size means a value measured by a scattering type particle size distribution measurement device, and an average particle size means an arithmetic average diameter calculated from the obtained particle size distribution.

The void ratio of the particles of the present invention can be appropriately set according to the desired use of the particles, and is, for example, in the range of 5 to 50%, preferably in the range of 10 to 50%, and more preferably in the range of 15 to 45%. Here, in the present invention, the void ratio means a value in which, using a cross-sectional image of the particle obtained by using a scanning electron microscope or the like, a ratio of a void area (a total of the area of the void portion in the cross-sectional image of the particle) is shown as a percentage when a cross-sectional area (an area of the entire cross section in the cross-sectional image of the particle) is made 100, and the average void ratio means an arithmetic average value of the obtained void ratio. Specifically, the void ratio of the particles of the present invention can be calculated according to Evaluation Example 5 mentioned later. It is also possible to similarly calculate the void ratio by using a means such as X-ray CT, or the like. By the void ratio of the particles of the present invention being in such a range, softness of the particles can be maintained and also excellent optical characteristics can be exhibited even in a foundation prescription.

A hardness of the particles of the present invention can be appropriately set according to the desired use of the particles, and is, for example, in the range of 0.1 to 5 MPa, preferably in the range of 0.1 to 4 MPa, and more preferably in the range of 0.5 to 3 MPa. Here, in the present invention, the hardness means a value measured by a microcompression testing machine, and is calculated from the following formula as the strength C(x) when the particle size is deformed by 10%.

$$C(x) = \frac{2.48P}{\pi d^2}$$

(wherein P represents a test force (N) when a particle size is deformed by 10%, π represents a ratio of the circumference of a circle to its diameter, d represents a particle size (mm), and C(x) represents a 10% strength (MPa).)

Specifically, the hardness of the particles of the present invention can be measured according to Evaluation Example 6 mentioned later. By the hardness of the particles of the present invention being in such a range, softness of the particles can be maintained and excellent optical characteristics can be exhibited.

(Light scattering rate)

The particles of the present invention are characterized in that a light scattering rate represented by the following equation (1) is in the range of 50 to 230%.

$$\text{Light scattering rate (\%)} = \frac{(\text{Reflection intensity at angle of } 20°/\cos 20°) + (\text{Reflection intensity at angle of } 70°/\cos 70°)}{2 \times (\text{Reflection intensity at angle of } 5°/\cos 5°)} \times 100$$

$$\cdots (1)$$

{in the formula (1), the reflection intensities at angles of 20°, 70° and 5° mean intensities of the reflected light at angles of 20°, 70° and 5° of a light receiver when light is incident from a certain angle after setting the sensitivity of the light receiver is set to an arbitrary value (which is referred to as a sensitivity adjustment value) when light is incident on particles at an angle of -30° with the normal direction to the particle being 0°.}

Here, the light scattering rate is calculated in accordance with the above-mentioned formula (1) described in WO 2010/092890. For example, when the light scattering rate at the incident angle of light is -30° is to be calculated, first, the normal direction of the surface against which the sample is pressed is 0°, and after the sensitivity of the light receiver at the time of incident of light to the sample from an angle of -30° is set to be an arbitrary value (which is referred to as a sensitivity adjustment value), the intensities of the reflected light at the angles of 20°, 70° and 5° of the light receiver are measured. Then, while maintaining the initial sensitivity adjustment value, the intensities of the reflected light at the angles of 20°, 70° and 5° of the light receiver at the time of incident of light to the same sample from an angle of -45° are measured. In the case of an incident angle of -60°, a relative intensity is similarly measured, and the light scattering rate is finally calculated.

When the light scattering rate represented by the above-mentioned formula (1) becomes 100%, the graph showing reflection intensity becomes circular, which means that the incident light is uniformly diffused. If this light scattering rate exceeds 100%, it means that the graph showing the reflection intensity becomes a horizontally long ellipse, and if it is less than 100, it means a vertically long ellipse.

The particles of the present invention have a reflected light intensity having higher uniformity despite of having an uneven structure on the surface thereof. The particles of the present invention are characterized in that the light scattering rate is in the range of 50 to 230%. At each observation of light incident angles of -30°, -45° and -60°, preferably the light scattering rate is in the range of 50 to 230% in either one of the incident angles, more preferably the light scattering rate is in the range of 50 to 230% in arbitrary two incident angles, and further preferably the light scattering rate is in the range of 50 to 230% in all of the incident angles. The light scattering rate of the particles of the present invention is more preferably in the range of 70 to 230%, and further preferably in the range of 90 to 230%. This means that the particles of the present invention can enable more uniform omnidirectional reflection and, for example, when the particles of the present invention are used as an additive for cosmetics, it can be expected to develop a defocus effect.

In particular, in a measurement of distribution of the reflected light, it is preferable that a ratio of the maximum intensity of the reflected light in the vicinity of specular reflection (for example, within an angle of specular reflection $\pm$ 10°, preferably within an angle of specular reflection $\pm$ 5°) to the incident light, to the maximum intensity of the reflected light in the vicinity of the incident light (for example, within an angle of incident light $\pm$ 10°, preferably within an angle of incident light $\pm$ 5°) is in the range of 0.5 : 1 to 2 : 1, and preferably in the range of 0.5 : 1 to 1.8 : 1. When the intensity of specular reflection is in such a range, it can be expected to exhibit excellent optical characteristics, in particular, the defocus effect when the particles of the present invention are used as an additive for cosmetics (in particular, an additive for a foundation).

<Method for producing particles>

The particles of the present invention can be produced by a method which comprises a step of obtaining a dispersion of the cellulose or the cellulose derivative and the clay mineral, and a step of spray-drying the obtained dispersion.

In the dispersion according to the producing method of the present invention, examples of the cellulose or the cellulose derivative and the clay mineral, and preferred embodiments are as mentioned above. The dispersion can be prepared by an optional method and, for example, can be obtained by mixing the cellulose or the cellulose derivative, the clay mineral and a dispersing medium, and subjecting the mixture to a pulverization treatment. Or else, the cellulose or the cellulose derivative (or the clay mineral) and a dispersing medium are firstly mixed, the mixture is subjected to a pulverization treatment to obtain a cellulose or a cellulose derivative (or a clay mineral) dispersion, then, the clay mineral (or the cellulose or the cellulose derivative) and a dispersing medium are mixed, and further the mixture is subjected to a pulverization treatment to obtain the dispersion. The dispersing medium is preferably an aqueous medium, and more preferably water, a water miscible organic solvent or a mixture thereof. Examples of the water miscible organic solvent may be mentioned an alcohol having 1 to 4 carbon atoms such as methanol, ethanol, isopropyl alcohol, butanol, etc., a ketone such as acetone, etc., a nitrile such as acetonitrile, etc., an amide such as N-methylpyrrolidone, N-cyclohexylpyrrolidone, N,N-dimethylacetamide, N,N-dimethylformamide, etc., a lactone such as $\gamma$-butyrolactone, etc., and an ether such as tetrahydrofuran, etc. In the most suitable embodiment, the dispersing medium is water, or a mixture of water and an alcohol having 1 to 4 carbon atoms.

The dispersion contains 0.1 to 20 parts by mass of the clay mineral with respect to 1 part by mass of the cellulose or the cellulose derivative, preferably 0.2 to 20 parts by mass, more preferably 0.5 to 15 parts by mass, and particularly preferably 1 to 10 parts by mass. Also, a concentration of the solid components including the cellulose or the cellulose derivative and the clay mineral in the dispersion is not particularly limited as long as it is in the range capable of being used in the subsequent spray-drying step and, for example, it is 0.5 to 20% by mass, preferably 1 to 15% by mass, and more preferably 2 to 10% by mass.

An operation of obtaining the dispersion is not particularly limited, and can be carried out using the known operation for obtaining the dispersion known to those skilled in the art. Typically, the dispersion can be obtained by pulverizing treatment of the cellulose or the cellulose derivative and the clay mineral, preferably obtained by physical pulverization. The physical pulverization is carried out by applying a physical external force to a mixture of the cellulose or the cellulose derivative and/or the clay mineral and a dispersing medium using a stirring apparatus such as a magnetic stirrer, a stirring blade, etc., a homogenizer such as a polytron, etc., an ultrasonic generating apparatus such as an ultrasonic crusher, etc., a pulverizer such as a wet-type pulverization apparatus (for example, Star Burst; SUGINO MACHINE LIMITED), etc. However, if the commercially available cellulose or the cellulose derivative and/or the clay mineral is/are a material(s) sufficiently pulverized, the dispersion may be obtained without carrying out the pulverization treatment. Also, in the producing method of the present invention, a commercially available cellulose dispersion, for example, a dispersion of a commercially available cellulose nanofiber may be used in place of the step of obtaining the cellulose or cellulose derivative dispersion.

The particles of the present invention are obtained by subjecting the obtained dispersion to spray-drying. Spray-drying is carried out using a known spray-drying device such as an atomizer, a spray dryer, a micro mist spray dryer, etc. Spray-drying conditions are appropriately set depending on the kind of the dispersing medium in the dispersion, the kind or the concentration of the cellulose or the cellulose derivative, etc., and, for example, are carried out at an inlet temperature of 150 to 300°C and an outlet temperature of 0 to 150°C.

<Cosmetics>

The particle of the present invention has an uneven structure on the surface thereof, and has an appropriate hardness and void ratio so that it is soft, and has excellent optical characteristics (light scattering properties) that the incident light is uniformly light scattered, so that it is suitable for adding to cosmetics that directly touch the skin and require optical properties such as a defocus effect. Examples of such cosmetics may be mentioned toiletry products such as face wash foams, face wash powders, body wash products, etc., hair care products such as shampoos, conditioners, etc., oral care products such as dentifrices, etc., makeup cosmetics such as makeup bases, powder foundations, liquid foundations, BB creams, concealer, sunscreen, etc., and these can be used as a scrubbing agent for enhancing a massage effect or a cleaning effect, or as a light scattering agent for expressing a defocusing effect, etc.

EXAMPLES

[Reference Synthetic Example 1: 2% by mass microcrystalline cellulose dispersion]

Into a wet-type pulverization apparatus Star Burst (manufactured by SUGINO MACHINE LIMITED) charged with 1,600 g of water subjecting to a pressure treatment with 220 MPa was added 400 g of a 10% by mass suspension of microcrystalline cellulose (Comprecel M101, Fushimi Pharmaceutical Co., Ltd.) with each about 10 g/min. When the concentration of the microcrystalline cellulose became 2% by mass, the mixture was subjected to pulverization treatments with 19 times at a pressure of 220 MPa to obtain the title microcrystalline cellulose dispersion.

[Example 1: Particles of cellulose : talc = 2 : 1 w/w]

A mixed liquid comprising 200 g of the microcrystalline cellulose dispersion obtained in Reference Synthetic Example 1 and 200 g of 1% by mass aqueous dispersion of talc (available from Nippon Talc Co., Ltd.) was stirred and spray-dried at an atomizer rotation speed of 20,000 rpm, an inlet temperature of 160°C, and a cyclone differential pressure of 0.5 kPa to obtain 3.5 g of powder as the title particles.

[Example 2: Particles of cellulose : talc = 1 : 2 w/w]

A mixed liquid comprising 200 g of the microcrystalline cellulose dispersion obtained in Reference Synthetic Example 1 and 200 g of 4% by mass aqueous dispersion of talc (available from Nippon Talc Co., Ltd.) was stirred and spray-dried at an atomizer rotation speed of 20,000 rpm, an inlet temperature of 160°C, and a cyclone differential pressure of 0.5 kPa to obtain 5.0 g of powder as the title particles.

[Example 3: Particles of cellulose : talc = 1 : 4.5 w/w]

A mixed liquid comprising 200 g of the microcrystalline cellulose dispersion obtained in Reference Synthetic Example 1 and 200 g of 9% by mass aqueous dispersion of talc (available from Nippon Talc Co., Ltd.) was stirred and spray-dried at an atomizer rotation speed of 20,000 rpm, an inlet temperature of 160°C, and a cyclone differential pressure of 0.5 kPa to obtain 11.3 g of powder as the title particles.

[Example 4: Particles of cellulose : talc = 1 : 2 w/w]

A mixed liquid comprising 200 g of the microcrystalline cellulose dispersion obtained in Reference Synthetic Example 1 and 200 g of 4% by mass aqueous dispersion of talc (available from ASADA MILLING CO., LTD.) was stirred and spray-dried at an atomizer rotation speed of 20,000 rpm, an inlet temperature of 160°C, and a cyclone differential pressure of 0.5 kPa to obtain 6.4 g of powder as the title particles.

[Reference Synthetic Example 2: 4% by mass microcrystalline cellulose dispersion]

Microcrystalline cellulose (Comprecel M101) was sieved with an opening of 106 $\mu$m to obtain 60 g of passed powder. Then, after the powder was dispersed in 1,440 g of pure water, the dispersion was subjected to pulverization treatments 6 times with a wet-type pulverization apparatus Star Burst (manufactured by SUGINO MACHINE LIMITED) at 200 MPa to obtain the titled 4% by mass microcrystalline cellulose dispersion.

[Example 5: Particles of cellulose : talc = 1 : 4 w/w]

A mixture comprising 225 g of the microcrystalline cellulose dispersion obtained in Reference Synthetic Example 2, 36 g of talc (available from Nippon Talc Co., Ltd.), and 639 g of pure water was subjected to homogenization treatment at a stirring rate of 10,000 rpm to obtain a dispersion. Spray-drying was carried out using the obtained dispersion at an atomizer rotation speed of 20,000 rpm, an inlet temperature of 160°C, and a cyclone differential pressure of 0.5 kPa to obtain 34 g of powder as the title particles.

[Example 6: Particles of cellulose : talc = 1 : 4 w/w]

A mixture comprising 200 g of the microcrystalline cellulose dispersion obtained in Reference Synthetic Example 2, 32 g of talc (available from Nippon Talc Co., Ltd.), and 168 g of pure water was subjected to homogenization treatment at a stirring rate of 10,000 rpm to obtain a dispersion. Spray-drying was carried out using the obtained dispersion at an atomizer rotation speed of 20,000 rpm, an inlet temperature of 160°C, and a cyclone differential pressure of 0.5 kPa to obtain 17 g of powder as the title particles.

[Example 7: Particles of cellulose : kaolin =1: 4 w/w]

A mixture comprising 100 g of the microcrystalline cellulose dispersion obtained in Reference Synthetic Example 2, 16 g of kaolin (Shokozan Mining Co., Ltd., pharmacopeia grade), and 284 g of pure water was subjected to homogenization treatment at a stirring rate of 10,000 rpm to obtain a dispersion. Spray-drying was carried out using the obtained dispersion at an atomizer rotation speed of 20,000 rpm, an inlet temperature of 160°C, and a cyclone differential pressure of 0.5 kPa to obtain 17 g of powder as the title particles.

[Example 8: Particles of cellulose : sericite =1 : 1 w/w]

A dispersion comprising a mixture of 400 g of the microcrystalline cellulose dispersion obtained in Reference Synthetic Example 2, 16 g of sericite (SoftSericite T-6, available from Dainihonkasei Co., Ltd.), and 864 g of pure water was obtained. Spray-drying was carried out using the obtained dispersion at an atomizer rotation speed of 15,000 rpm, an inlet temperature of 150°C, and a cyclone differential pressure of 0.5 kPa to obtain 17 g of powder as the title particles.

[Example 9: Particles of cellulose : sericite =1 : 4 w/w]

A dispersion comprising a mixture of 200 g of the microcrystalline cellulose dispersion obtained in Reference Synthetic Example 2, 32 g of sericite (SoftSericite T-6, available from Dainihonkasei Co., Ltd.), and 1,368 g of pure water was obtained. Spray-drying was carried out using the obtained dispersion at an atomizer rotation speed of 15,000 rpm, an inlet temperature of 150°C, and a cyclone differential pressure of 0.5 kPa to obtain 21 g of powder as the title particles.

[Example 10: Particles of cellulose : talc = 1 : 4 w/w]

A dispersion comprising a mixture of 250 g of the microcrystalline cellulose dispersion obtained in Reference Synthetic Example 2, 40 g of talc (available from Nippon Talc Co., Ltd.), and 210 g of pure water was obtained. Spray-drying was carried out using the obtained dispersion under the conditions of an inlet temperature of 160°C, a blower of 0.34 m$^3$/min, and atomizing of 100 kPa and using EYELA SPRAY DRYER SD-1000 (manufactured by TOKYO RIKAKIKAI CO., LTD.) to obtain 5 g of powder as the title particles.

[Reference Synthetic Example 3: 10% by mass microcrystalline cellulose dispersion]

In 16.2 kg of ion exchanged water was dispersed 1.8 kg of the microcrystalline cellulose (Comprecel M101), and then, the dispersion was subjected to pulverization treatments 3 times with a wet-type pulverization apparatus Star Burst (manufactured by SUGINO MACHINE LIMITED) at 245 MPa to obtain the titled 10% by mass microcrystalline cellulose dispersion.

[Example 11: Particles of cellulose : talc = 1 : 2 w/w]

A dispersion comprising a mixture of 200 g of the microcrystalline cellulose dispersion obtained in Reference Synthetic Example 3, 40 g of talc (available from Nippon Talc Co., Ltd.), and 360 g of pure water was obtained. The obtained dispersion was sprayed by mounting an SE nozzle on MDL-050B (manufactured by Fujisaki Denki K.K.) at an inlet temperature of 160°C, a supply air volume of 1.00 m$^3$/min, a nozzle air flow amount of 40 NL/min, and a liquid amount of 8 mL/min to obtain 33 g of powder as the title particles.

[Example 12: Particles of cellulose : talc = 1 : 4 w/w]

A dispersion comprising a mixture of 120 g of the microcrystalline cellulose dispersion obtained in Reference Synthetic Example 3, talc (available from Nippon Talc Co., Ltd.)48g, and 432 g of pure water was obtained. The obtained dispersion was sprayed by mounting an SE nozzle on MDL-050B (manufactured by Fujisaki Denki K.K.) at an inlet temperature of 160°C, a supply air volume of 1.00 m$^3$/min, a nozzle air flow amount of 40 NL/min, and a liquid amount of 8 mL/min to obtain 39 g of powder as the title particles.

[Example 13: Particles of cellulose : talc = 1 : 9 w/w]

A dispersion comprising a mixture of 60 g of the microcrystalline cellulose dispersion obtained in Reference Synthetic Example 3, 54 g of talc (available from Nippon Talc Co., Ltd.), and 486 g of pure water was obtained. The obtained dispersion was sprayed by mounting an SE nozzle on MDL-050B (manufactured by Fujisaki Denki K.K.) at an inlet temperature of 160°C, a supply air volume of 1.00 m$^3$/min, a nozzle air flow amount of 40 NL/min, and a liquid amount of 8 mL/min to obtain 55 g of powder as the title particles.

[Example 14: Particles of cellulose : talc = 1 : 4 w/w]

A dispersion comprising a mixture of 120 g of the microcrystalline cellulose dispersion obtained in Reference Synthetic Example 3, 48 g of talc (available from Nippon Talc Co., Ltd.), and 282 g of pure water was obtained. The obtained dispersion was sprayed by mounting an SE nozzle on MDL-050B (manufactured by Fujisaki Denki K.K.) at an inlet temperature of 160°C, a supply air volume of 1.00 m$^3$/min, a nozzle air flow amount of 40 NL/min, and a liquid amount of 8 mL/min to obtain 33 g of powder as the title particles.

[Comparative Example 1: Particles of cellulose alone]

In 750 g of pure water was dispersed 75 g of the microcrystalline cellulose (Comprecel M101). This dispersion was subjected to pulverization treatment 30 times by a wet-type pulverization apparatus Star Burst (manufactured by SUGINO MACHINE LIMITED) with a pressure of 220 MPa to obtain a crystalline cellulose dispersion. Ten grams of the obtained dispersion was weighed in a petri dish, and subjected to drying at 105°C for 1 hour to remove the water content. The amount of the obtained residue was measured, and the cellulose concentration (solid content concentration) in the obtained crystalline cellulose dispersion was calculated from the weight difference before and after drying. As a result, the concentration was 4.0% by mass. To 250 g of the crystalline cellulose dispersion was added 250 g of pure water to dilute the dispersion to obtain a 2.0% by mass crystalline cellulose dispersion, and the dispersion was spray-dried at an atomizer rotation speed of 20,000 rpm, an inlet temperature of 160°C, and a cyclone differential pressure of 0.5 kPa for 23 minutes to obtain 4.6 g of powder as the title particles. During the procedure, the outlet temperature changed between 109°C and 95°C.

[Evaluation Example 1: Evaluation of particle size of cellulose particles]

Using a scattering type particle size distribution measuring device LA-960 (manufactured by HORIBA Ltd.), concentrations of the sample solutions of each particle obtained in Examples 1 to 14 were adjusted various kinds of the sample concentrations were adjusted so that the transmittance of a semiconductor laser (650 nm) and a light emitting diode (405 nm) became 90.0% or less with the state that the sample solution was circulated with a circulation rate of "3" and a stirring rate of "2". To the obtained sample solutions were irradiated ultrasonic waves at an ultrasonic wave intensity "3" for 1 minute, and then the particle size measurement was carried out. The results are shown in FIG. 1(a) to (n) in order.

[Evaluation Example 2: Evaluation of average frictional coefficient and fluctuation of average frictional coefficient]

0.3 g of each sample was weighed, and the frictional coefficient and fluctuation of frictional coefficient were evaluated by a friction tester KES-SE (manufactured by KATO TECH CO., LTD.) equipped with a 10 mm square silicon material sensor. Measurements of each sample were carried out 5 times. As the samples, particles obtained in Comparative Example 1, and Examples 2, 3, 5 and 7, and Toray Nylon fine particles SP-500 (manufactured by TORAY INDUSTRIES, INC.) as a control were used. Here, as the sensor, 10 mm square silicon wire was used, and a measurement distance was set to 20 mm, a static load was 25 gf, a measurement speed was 1.0 mm/sec, and a contact surface width was 10 mm. The results were shown in FIG. 2 and FIG. 3.

In FIG. 2, MIU refers to an average frictional coefficient, which is an index showing the slipperiness felt when touching the surface of an object with a human finger. When the value of MIU is smaller, it is easier to slip, and the value is larger, it is less slippery.

In FIG. 3, MMD refers to fluctuation of an average frictional coefficient, which is an index showing the smoothness and roughness felt when touching the surface of an object with a human finger. When the value of MMD is smaller, it is smoother, and the value is larger, it feels more roughness.

[Evaluation Example 3: Measurement of distribution of reflected light]

A black drawing paper PI-N86D (manufactured by Maruai Inc.) was pasted onto a micro slide glass S1111 (manufactured by Matsunami Glass Ind., Ltd.) using NICETACK (Registered Trademark) NW-10S (manufactured by Nichiban Co., Ltd.). Then, NICETACK (Registered Trademark) NW-10S was pasted on the above-mentioned black drawing paper, and after pressing the sample powder thereon, excess powder was removed by an air gun adjusted to a pressure of 0.2 MPa. Measurement of the distribution of the reflected light was carried out by a goniophotometer GP-5 (manufactured by Murakami Color Research Laboratory Co., Ltd.). The measurement incident light was carried out at three points of -30, -45 and -60°. The light scattering rate at each angle of the incident light was calculated in accordance with the following equation (1) (see WO 2010/092890).

$$\text{Light scattering rate } (\%) = \frac{(\text{Reflection intensity at angle of } 20^\circ / \cos 20^\circ) + (\text{Reflection intensity at angle of } 70^\circ / \cos 70^\circ)}{2 \times (\text{Reflection intensity at angle of } 5^\circ / \cos 5^\circ)} \times 100$$

$$\cdots \quad (1)$$

As the samples, particles obtained in Examples 1 to 4, and particles obtained in Examples 7 to 14 were used. The reflection intensity and the light scattering rate of the samples for each incident light were shown in Tables 1 to 3. In addition, the correlation between the angle of the incident light and the light scattering rate of each sample was shown in Table 4 and FIGs. 4(a) and (b).

[Table 1]

| Results at incident light of -30° | | | | |
|---|---|---|---|---|
| Evaluated sample | Reflection intensity at angle of 5° | Reflection intensity at angle of 20° | Reflection intensity at angle of 70° | Light scattering rate (%) |
| Example 1 | 68.49 | 68.80 | 34.77 | 127.18 |
| Example 2 | 78.91 | 76.59 | 36.57 | 118.94 |
| Example 3 | 72.28 | 71.21 | 33.23 | 119.18 |
| Example 4 | 56.08 | 54.59 | 24.72 | 115.79 |
| Example 7 | 51.66 | 50.75 | 24.56 | 121.31 |
| Example 8 | 51.38 | 50.50 | 26.45 | 127.07 |
| Example 9 | 51.30 | 50.24 | 26.11 | 126.03 |
| Example 10 | 51.27 | 50.19 | 25.73 | 124.98 |

(continued)

| | Results at incident light of -30° | | | |
|---|---|---|---|---|
| Evaluated sample | Reflection intensity at angle of 5° | Reflection intensity at angle of 20° | Reflection intensity at angle of 70° | Light scattering rate (%) |
| Example 11 | 51.26 | 50.67 | 25.03 | 123.51 |
| Example 12 | 51.40 | 51.87 | 29.05 | 135.80 |
| Example 13 | 51.62 | 52.26 | 25.27 | 124.96 |
| Example 14 | 51.33 | 51.41 | 27.81 | 131.99 |

[Table 2]

| | Results at incident light of -45° | | | |
|---|---|---|---|---|
| Evaluated sample | Reflection intensity at angle of 5° | Reflection intensity at angle of 20° | Reflection intensity at angle of 70° | Light scattering rate (%) |
| Example 1 | 68.61 | 69.45 | 41.24 | 141.19 |
| Example 2 | 77.90 | 76.34 | 42.43 | 131.27 |
| Example 3 | 71.81 | 69.56 | 36.78 | 125.94 |
| Example 4 | 55.49 | 53.57 | 27.24 | 122.66 |
| Example 7 | 51.35 | 50.30 | 27.48 | 129.86 |
| Example 8 | 51.83 | 51.92 | 31.67 | 142.09 |
| Example 9 | 51.65 | 53.31 | 35.46 | 154.69 |
| Example 10 | 51.15 | 50.89 | 30.72 | 140.20 |
| Example 11 | 50.77 | 51.15 | 31.22 | 142.96 |
| Example 12 | 51.77 | 53.97 | 37.79 | 161.57 |
| Example 13 | 50.15 | 51.49 | 32.77 | 149.59 |
| Example 14 | 51.65 | 53.31 | 35.46 | 154.69 |

[Table 3]

| | Results at incident light of -60° | | | |
|---|---|---|---|---|
| Evaluated sample | Reflection intensity at angle of 5° | Reflection intensity at angle of 20° | Reflection intensity at angle of 70° | Light scattering rate (%) |
| Example 1 | 65.17 | 67.42 | 52.84 | 172.92 |
| Example 2 | 72.67 | 72.83 | 51.44 | 156.21 |
| Example 3 | 68.58 | 67.28 | 42.00 | 141.19 |
| Example 4 | 52.46 | 51.51 | 32.77 | 143.02 |

(continued)

| Results at incident light of -60° | | | |
|---|---|---|---|
| Evaluated sample | Reflection intensity at angle of 5° | Reflection intensity at angle of 20° | Reflection intensity at angle of 70° | Light scattering rate (%) |
| Example 7 | 49.03 | 48.94 | 32.58 | 149.68 |
| Example 8 | 49.47 | 50.93 | 39.97 | 172.24 |
| Example 9 | 49.16 | 52.63 | 50.31 | 205.79 |
| Example 10 | 49.02 | 50.83 | 49.29 | 201.40 |
| Example 11 | 48.71 | 52.78 | 54.99 | 221.85 |
| Example 12 | 46.24 | 48.94 | 52.73 | 222.18 |
| Example 13 | 49.16 | 52.63 | 50.31 | 205.79 |
| Example 14 | 45.58 | 49.76 | 39.93 | 185.45 |

[Table 4]

| Correlation between angle of the incident light and light scattering rate | | | |
|---|---|---|---|
| Evaluated sample | Light scattering rate at incident light of -30° | Light scattering rate at incident light of -45° | Light scattering rate at incident light of -60° |
| Example 1 | 127.18 | 141.19 | 172.92 |
| Example 2 | 118.94 | 131.27 | 156.21 |
| Example 3 | 119.18 | 125.94 | 141.19 |
| Example 4 | 115.79 | 122.66 | 143.02 |
| Example 7 | 121.31 | 129.86 | 149.68 |
| Example 8 | 127.07 | 142.09 | 172.24 |
| Example 9 | 126.03 | 154.69 | 205.79 |
| Example 10 | 124.98 | 140.20 | 185.45 |
| Example 11 | 123.51 | 142.96 | 201.40 |
| Example 12 | 135.80 | 161.57 | 221.85 |
| Example 13 | 124.96 | 149.59 | 222.18 |
| Example 14 | 131.99 | 154.69 | 205.79 |

A drawing of the distribution of the reflected light of the particles obtained in Example 2 at an incident light of -45° is shown in FIG. 5(a). Also, as Comparative Example, a drawing of the distribution of the reflected light of CELLULOBEADS D-10 (Daito Kasei Kogyo Co., Ltd.) at an incident light of -45° was shown in FIG. 5(b). In FIG. 5(b), the graph showing the reflection intensity is greatly distorted, and the intensity of the reflected light in the vicinity of the specular reflection with respect to the incident light is significantly larger than that of the other reflection intensities (in particular, the reflected light in the vicinity of the incident light), so that the soft focus effect cannot be expected. On the other hand, in FIG. 5(a), the graph showing the reflection intensity shows an elliptical shape close to a circle, and the intensity of the reflected light in the vicinity of the specular reflection with respect to the incident light is the same degree of the intensities of the other reflected lights (in particular, the reflected light in the vicinity of the incident light), so that a soft focus effect can be expected.

[Evaluation Example 4: Residue on ignition test]

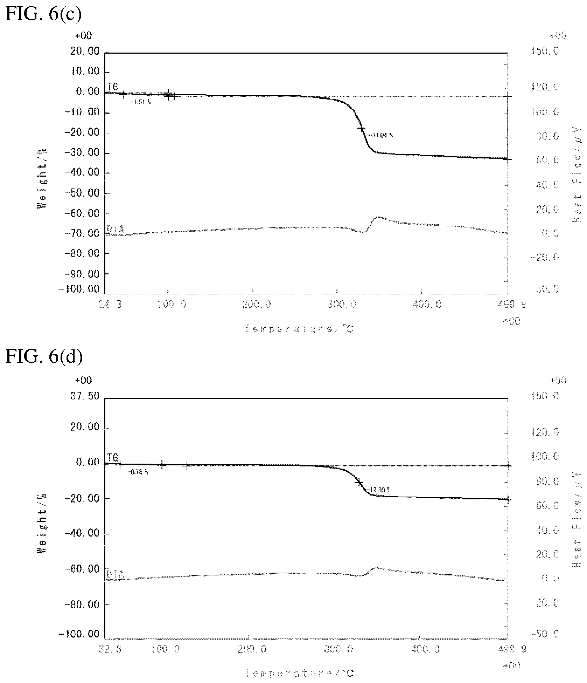

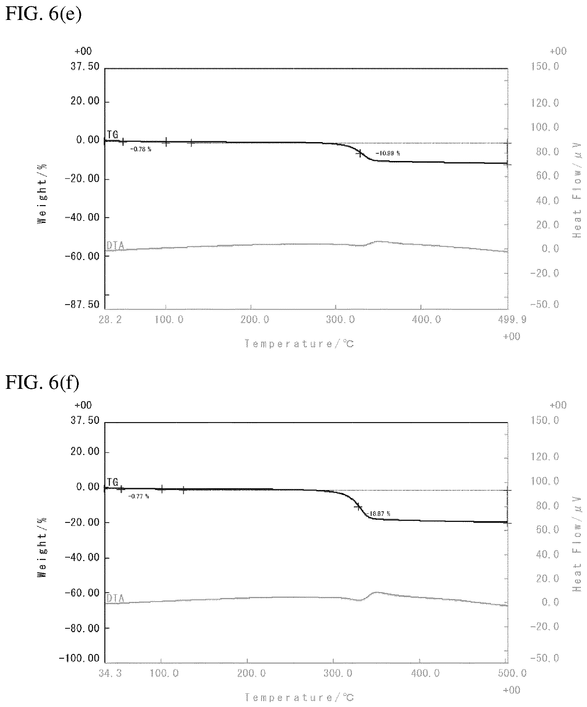

Using the particles obtained in Examples 1, 2, and 11 to 14, thermogravimetry-differential thermal analysis (TG-DTA) was carried out. Change in weight and change in calories were measured using a thermal analyzer Rigaku Thermoplus EVO TG8120 (manufactured by Rigaku Corporation), and using a pan made of aluminum as a sample container and about 5 mg of aluminum oxide as a standard substance, under an air atmosphere, by elevating a sample amount of 5 mg with a temperature raising rate of 10.0°C/min. The measurement results of each particle were shown in FIG. 6(a) to (f) in order. In FIG. 6, the weight loss of less than 100°C is due to the water content contained in the powder, and the weight loss of around 300°C is due to thermal decomposition of cellulose.

[Evaluation Example 5: Calculating methods of cross section and void ratio of particles]

After coating a silver paste on a silicon wafer, the particles obtained in Example 12 were sprinkled. Then, after removing the excess sample by air blow, platinum was vapor-deposited at 15 mA for 100 seconds using ion sputtering MC1000 (manufactured by Hitachi, Ltd.) to prepare a sample. A cross section of this sample was prepared using a focused ion beam-scanning electron microscope (FIB-SEM) Helios NanoLab G3 (manufactured by Thermo Fisher Scientific) at an acceleration voltage of 1 kV and a current value of 0.1 nA, and observation was carried out. The reflected electron image obtained from the observation was analyzed using Avizo 9.5 software (manufactured by Thermo Fisher Scientific), and the void ratio was calculated according to the following equation. Here, the test was carried out twice.

$$Void\ ratio\ (\%) = \frac{Void\ area}{Cross\text{-}sectional\ area} \times 100\%$$

(wherein the void area indicates the total area of the void portion in the cross-sectional image of the particle, and the cross-sectional area indicates the area of the entire cross section in the cross-sectional image of the particle.)

The cross-sectional observation image of the particles obtained in Example 12 was shown in FIG. 7(a), the image highlighting the entire cross section of particles used for calculating the void ratio and its area (cross-sectional area) were shown in FIG. 7(b), and the image highlighting the void portion of the entire cross section of particles and its area (cross-sectional area) were shown in FIG. 7(c). As a result, the void ratio was 19.90%. With regard to the other particles obtained in Example 12, the void ratio was calculated by the same manner and as a result, it was 23.30%, and an average value of the void ratio was 21.60%. With regard to the particles obtained in Examples 1, 5, and 8 to 13, the void ratio and an average value thereof were calculated according to the same manner. The average value of the void ratios of each particle was shown in Table 5.

[Table 5]

| Evaluated sample | Void ratio (%) | | |
|---|---|---|---|
| | 1st time | 2nd time | Average value |
| Example 1 | 4.20 | 7.50 | 5.90 |
| Example 5 | 15.70 | 22.00 | 18.80 |
| Example 8 | 36.50 | 38.20 | 37.40 |
| Example 9 | 44.10 | 44.30 | 44.20 |
| Example 10 | 19.90 | 15.80 | 17.90 |
| Example 11 | 15.60 | 18.70 | 17.20 |
| Example 12 | 19.90 | 23.30 | 21.60 |
| Example 13 | 16.80 | 25.50 | 21.20 |

[Evaluation Example 6: Measurement of hardness of particles]

Using a microcompression testing machine MCT-510 (manufactured by Shimadzu Corporation), hardness of the particles obtained in Examples 1, 5, 6, and 8 to 13 were measured. The measurement was carried out with specifications of a test force of 49.00 mN, a load speed of 0.4462 mN/sec, and an upper pressing indenter of 50 $\mu$m or 20 $\mu$m. An extremely minor amount of the sample was sprayed on a lower pressure plate, and the compression test was carried out particle by particle. A particle hardness was calculated from the following equation as the strength C(x) when the particle size was deformed by 10%. The test was carried out 5 times, and the average value was calculated. The particle size was measured with a length measurement kit attached to the device.

$$C(x) = \frac{2.48P}{\pi d^2}$$

(wherein P represents the test force (N) when the particle size is deformed by 10%, $\pi$ represents a ratio of the circumference of a circle to its diameter, d represents a particle size (mm), and C(x) represents a 10% strength (MPa).)

An average particle size, a test force at the time of 10% deformation, and 10% strength of each sample are shown in Table 6.

[Table 6]

| Evaluated sample | Average particle size (μm) | Test force at 10% deformation (mN) | 10% strength C(x) (MPa) |
|---|---|---|---|
| Example 1 | 18.97 | 1.90 | 4.13 |
| Example 5 | 21.37 | 0.36 | 0.63 |
| Example 6 | 21.44 | 0.94 | 1.61 |
| Example 8 | 20.91 | 1.46 | 2.62 |
| Example 9 | 20.85 | 0.46 | 0.84 |
| Example 10 | 11.03 | 0.07 | 0.46 |
| Example 11 | 6.63 | 0.03 | 0.60 |
| Example 12 | 6.52 | 0.03 | 0.60 |
| Example 13 | 6.35 | 0.03 | 0.52 |

[Evaluation Example 7: Measurement of distribution of reflected light in rouge]

In a microtube natural (manufactured by AS ONE Corporation) having a volume of 1.5 mL were weighed 45 mg of rouge (red iron oxide) (manufactured by Pinoa Co., Ltd.) and 5 mg of each powder obtained in Comparative Example 1, Examples 5 and 11 to 13, and the mixture was shaken and stirred by VORTEX GENIUS 3 (manufactured by IKA) for 5 minutes and mixed to prepare a rouge powder sample containing 10% of each particle.

A black drawing paper PI-N86D (manufactured by Maruai Inc.) was pasted onto a micro slide glass S1111 (manufactured by Matsunami Glass Ind., Ltd.) using NICETACK (Registered Trademark) NW-10S (manufactured by Nichiban Co., Ltd.). Then, NICETACK (Registered Trademark) NW-10S was pasted on the above-mentioned black drawing paper, and after pressing the sample powder thereon, excess powder was removed by an air gun adjusted to a pressure of 0.2 MPa. Measurement of the distribution of the reflected light was carried out by a goniophotometer GP-5 (manufactured by Murakami Color Research Laboratory Co., Ltd.). The measured incident light was made -45°, and the intensity of the reflected light at 45° was measured. The intensity of the reflected light of each sample powder of Comparative Example 1 or Example 5 was shown in Table 7, and the intensity of the reflected light of each sample powder of Examples 11 to 13 was shown in Table 8. Here, the intensity of the reflected light of rouge alone was also measured as a control.

[Table 7]

| Reflective strength of 45° at incident light -45° | |
|---|---|
| Rouge alone | 44.90 |
| Rouge powder containing 10% of particles of Comparative Example 1 | 43.50 |
| Rouge powder containing 10% of particles obtained in Example 5 | 41.50 |

[Table 8]

| Reflective strength of 45° at incident light -45° | |
|---|---|
| Rouge alone | 50.88 |
| Rouge powder containing 10% of particles obtained in Example 11 | 47.64 |
| Rouge powder containing 10% of particles obtained in Example 12 | 50.07 |
| Rouge powder containing 10% of particles obtained in Example 13 | 46.37 |

[Evaluation Example 8: Measurement of distribution of reflected light in talc]

In a microtube natural (manufactured by AS ONE Corporation) having a volume of 1.5 mL were each weighed 45 mg of talc (available from Nippon Talc Co., Ltd.) and 5 mg of the particles obtained in Example 5, and the mixture was shaken and stirred by VORTEX GENIUS 3 (manufactured by IKA) for 5 minutes and mixed to prepare a talc powder sample containing 10% of the particles.

A black drawing paper PI-N86D (manufactured by Maruai Inc.) was pasted onto a micro slide glass S1111 (manufactured by Matsunami Glass Ind., Ltd.) using NICETACK (Registered Trademark) NW-10S (manufactured by Nichiban Co., Ltd.). Then, NICETACK (Registered Trademark) NW-10S was pasted on the above-mentioned black drawing paper, and after pressing the sample powder thereon, excess powder was removed by an air gun adjusted to a pressure of 0.2 MPa. Measurement of the distribution of the reflected light was carried out by a goniophotometer GP-5 (manufactured by Murakami Color Research Laboratory Co., Ltd.). The measured incident light was made -45°, and the intensity of the reflected light at 45° was measured. The intensity of the reflected light of Example 5 was shown in Table 9. Here, the intensity of the reflected light of talc alone was also measured as a control.

[Table 9]

| Reflective strength of 45° at incident light -45° | |
|---|---|
| Talc alone | 57.87 |
| Talc containing 10% of particles obtained in Example 5 | 52.25 |

[Evaluation Example 9: Measurement of distribution of reflected light in sericite]

In a microtube natural (manufactured by AS ONE Corporation) having a volume of 1.5 mL were each weighed 45 mg of sericite (SoftSericite T-6, available from Dainihonkasei Co., Ltd.) and 5 mg of the particles obtained in Example 5, and the mixture was shaken and stirred by VORTEX GENIUS 3 (manufactured by IKA) for 5 minutes and mixed to prepare a sericite powder sample containing 10% of the particles.

A black drawing paper PI-N86D (manufactured by Maruai Inc.) was pasted onto a micro slide glass S1111 (manufactured by Matsunami Glass Ind., Ltd.) using NICETACK (Registered Trademark) NW-10S (manufactured by Nichiban Co., Ltd.). Then, NICETACK (Registered Trademark) NW-10S was pasted on the above-mentioned black drawing paper, and after pressing the sample powder thereon, excess powder was removed by an air gun adjusted to a pressure of 0.2 MPa. Measurement of the distribution of the reflected light was carried out by a goniophotometer GP-5 (manufactured by Murakami Color Research Laboratory Co., Ltd.). The measured incident light was made -45°, and the intensity of the reflected light at 45° was measured. The intensity of the reflected light of Example 5 was shown in Table 10. Here, the intensity of the reflected light of sericite alone was also measured as a control.

[Table 10]

| Reflective strength of 45° at incident light -45° | |
|---|---|
| Sericite alone | 61.20 |
| Sericite containing 10% of particles obtained in Example 5 | 51.22 |

[Evaluation Example 10: Measurement of distribution of reflected light in foundation prescription]

According to Table 11, in 50 mL of a vial was weighed Phase A and in 300 mL of a tall beaker was weighed Phase B, and each was heated and dissolved at about 75°C. To the above-mentioned heated Phase B was added Phase A, and the mixture was subjected to a QUICK HOMO MIXER (manufactured by Mizuho Kogyo Co., Ltd., 5,000 rpm, 3 minutes) treatment. Thereafter, the mixture was stirred and cooled with LABORATORY HIGH POWER MIXER (manufactured by AS ONE Corporation) until the liquid temperature reached about 35°C, and when the temperature became about 35°C, Phase C was added thereto and mixed by stirring. To a microtube STF-15 (manufactured by Sansyo Co., Ltd.) having a volume of 1.5 mL were each weighed 490 mg of the prepared oil in water (hereinafter abbreviated to as o/w.) foundation liquid and 10 mg of particle powder to be evaluated (Phase D), and the mixture was shaken and stirred by VORTEX GENIUS 3 (manufactured by IKA) for 5 minutes to prepare an o/w foundation sample containing 2% of each particle to be evaluated. As the particles to be evaluated, particles obtained in Examples 5, 9 and 11 to 13 were used. Also, as Comparative Examples, NYLON POWDER SP-500 (manufactured by TORAY INDUSTRIES, INC.) and CELLULOBEADS D-10 (manufactured by Daito Kasei Kogyo Co., Ltd.) were used.

[Table 11]

| Phase | Component | Composition |
|---|---|---|
| A | NIKKOL Nikkomulese 41[*1] | 2.00% |
| | NIKKOL NATURAL OILS-1[*1] | 7.00% |
| | NIKKOL Nikkoguard 88[*1] | 0.50% |
| B | Fresh Color base AQUA[*1] | 15.00% |
| | Purified water | 41.30% |
| | NIKKOL SMT[*1] | 0.10% |
| | EDTA-2Na[*2] | 0.10% |
| | 1% Hydroxypropyl cellulose water | 20.00% |
| | 1% Xanthan gum | 10.00% |
| c | NIKKOL NET-813-1[*1] | 2.00% |
| D | Particles to be evaluated | 2.00% |
| Total | | 100.00% |
| [*1]: available from Nikko Chemicals Co., Ltd. [*2]: KELCOGEL-CF-LA available from Sansho Co., Ltd. | | |

A black drawing paper PI-N86D (manufactured by Maruai Inc.) was pasted onto a micro slide glass S1111 (manufactured by Matsunami Glass Ind., Ltd.) using NICETACK (Registered Trademark) NW-10S (manufactured by Nichiban Co., Ltd.). Then, one drop of the sample o/w foundation was dropped onto the above-mentioned black drawing paper with a spuit, spread evenly, and air-dried. Measurement of the distribution of the reflected light was carried out by a goniophotometer GP-5 (manufactured by Murakami Color Research Laboratory Co., Ltd.). The measured incident light was made -45°, and the light scattering rate was calculated in accordance with the following equation (1). Also, intensity of the reflected light at 45° was measured. The results are shown in Table 12.
Moreover, the reflected light intensity at 45 ° was measured. The results are shown in Table 12.

$$\text{Light scattering rate } (\%) = \frac{(\text{Reflection intensity at angle of } 20°/\cos 20°) + (\text{Reflection intensity at angle of } 70°/\cos 70°)}{2 \times (\text{Reflection intensity at angle of } 5°/\cos 5°)} \times 100 \quad \cdots \quad (1)$$

[Table 12]

| Light scattering rate at incident light of -45° and reflection intensity at 45° | | | | | |
|---|---|---|---|---|---|
| Name of sample | Reflection intensity at angle of 5° | Reflection intensity at angle of 20° | Reflection intensity at angle of 70° | Light scattering rate (%) | Reflection intensity at angle of 45° |
| o/w foundation alone | 51.78 | 52.01 | 35.47 | 153.00 | 50.27 |

(continued)

| Light scattering rate at incident light of -45° and reflection intensity at 45° | | | | | |
|---|---|---|---|---|---|
| Name of sample | Reflection intensity at angle of 5° | Reflection intensity at angle of 20° | Reflection intensity at angle of 70° | Light scattering rate (%) | Reflection intensity at angle of 45° |
| o/w foundation containing 2% NYLON POWDER SP-500 alone | 50.87 | 49.86 | 31.09 | 140.96 | 45.53 |
| o/w foundation containing 2% of Comparative Example 1 | 51.34 | 49.83 | 29.17 | 134.19 | 43.82 |
| o/w foundation containing 2% of Example 5 | 51.16 | 49.56 | 27.96 | 130.94 | 42.97 |
| o/w foundation containing 2% of Example 11 | 51.27 | 49.78 | 27.84 | 130.55 | 43.23 |
| o/w foundation containing 2% of Example 12 | 51.2 | 49.74 | 27.8 | 130.57 | 43.26 |
| o/w foundation containing 2% of Example 13 | 50.98 | 49.19 | 27.35 | 129.28 | 42.57 |
| o/w foundation containing 2% of Example 9 | 50.91 | 49.37 | 28.12 | 131.84 | 43.28 |
| o/w foundation containing 2% of CELLULOBEADS D-10 | 51.23 | 49.85 | 29.18 | 134.53 | 44.04 |

UTILIZABILITY IN INDUSTRY

The particles of the present invention are natural material and soft, and are excellent in light scattering properties. Accordingly, it can be applied to the industrial field as a light diffusing agent or the like. In addition, the particles of the present invention has an uneven structure on the surface thereof (that is, since pores or voids are appropriately present), so that it is soft, and also has excellent optical characteristics (light scattering properties) that the incident light is uniformly light scattered, so that it can be expected to exhibit a defocus effect (also called a soft focus effect) whereby it is preferable to apply it to the field of cosmetics that comes into direct contact with the skin.

**Claims**

1.　A particle which comprises having an uneven structure on a surface thereof, and containing cellulose or a cellulose derivative and a clay mineral as main components.

2.　The particle according to Claim 1, which has a wrinkle-like or fold-like uneven structure on a surface thereof.

3.　The particle according to Claim 1 or 2, wherein a void ratio is in a range of 5 to 50%.

4.　The particle according to any one of Claims 1 to 3, wherein a particle size is in a range of 0.5 to 500 μm.

5.　The particle according to any one of Claims 1 to 4, wherein a hardness is in a range of 0.1 to 5 MPa.

6. The particle according to any one of Claims 1 to 5, wherein a light scattering rate is in a range of 50 to 230%.

7. The particle according to any one of Claims 1 to 6, wherein, in a measurement of a distribution of reflected light, a ratio of an intensity of the reflected light in a vicinity of a specular reflection to incident light with respect to an intensity of the reflected light in a vicinity of the incident light is in a range of 0.5: 1 to 2: 1.

8. The particle according to any one of Claims 1 to 7, wherein the particle contains 0.1 to 20 parts by mass of the clay mineral with respect to 1 part by mass of the cellulose or cellulose derivative.

9. The particle according to any one of Claims 1 to 8, wherein the cellulose is a crystalline cellulose.

10. The particle according to any one of Claims 1 to 9, wherein the clay mineral is at least one selected from the group consisting of talc, kaolin and sericite.

11. A method for producing a particle having an uneven structure on a surface thereof, and containing cellulose or a cellulose derivative and a clay mineral as main components, which comprises a step of obtaining a dispersion of cellulose or a cellulose derivative and a clay mineral, and a step of spray-drying the obtained dispersion.

12. The producing method according to Claim 11, wherein the dispersion is obtained by physical pulverization of the cellulose or the cellulose derivative and the clay mineral.

13. The producing method according to Claim 11 or 12, wherein a concentration of a solid content including the cellulose or the cellulose derivative and the clay mineral in the dispersion is 0.5 to 20% by mass.

14. The producing method according to any one of Claims 11 to 13, wherein the dispersion contains 0.1 to 20 parts by mass of the clay mineral with respect to 1 part by mass of the cellulose or the cellulose derivative.

15. A cosmetic which comprises the particle according to any one of Claims 1 to 10 or the particle obtained by the producing method according to any one of Claims 11 to 14.

FIG. 1(a)

FIG. 1(b)

FIG. 1(c)

FIG. 1(d)

FIG. 1(e)

FIG. 1(f)

FIG. 1(g)

FIG. 1(h)

FIG. 1(i)

FIG. 1(j)

FIG. 1(k)

FIG. 1(l)

FIG. 1(m)

FIG. 1(n)

FIG. 2

MIU

FIG. 3

MMD

FIG. 4

(a)

(b)

FIG. 5

( a )

( b )

FIG. 6(a)

FIG. 6(b)

FIG. 6(c)

FIG. 6(d)

FIG. 6(e)

FIG. 6(f)

FIG. 7

(a)

(b)

Entire area of particles : 14. 2244 ($\mu$ m²)

(c)

Sum of void area : 3. 3132 ($\mu$ m²)

<div align="center">

**INTERNATIONAL SEARCH REPORT**

</div>

| | International application No. |
|---|---|
| | PCT/JP2019/035935 |

**A. CLASSIFICATION OF SUBJECT MATTER**
Int.Cl. A61K8/25(2006.01)i, A61K8/02(2006.01)i, A61K8/73(2006.01)i, A61Q1/02(2006.01)i, A61Q1/12(2006.01)i

According to International Patent Classification (IPC) or to both national classification and IPC

**B. FIELDS SEARCHED**

Minimum documentation searched (classification system followed by classification symbols)
Int.Cl. A61K8/00-8/99, A61Q1/00-90/00

Documentation searched other than minimum documentation to the extent that such documents are included in the fields searched
Published examined utility model applications of Japan     1922-1996
Published unexamined utility model applications of Japan   1971-2019
Registered utility model specifications of Japan           1996-2019
Published registered utility model applications of Japan   1994-2019

Electronic data base consulted during the international search (name of data base and, where practicable, search terms used)
JSTPlus/JMEDPlus/JST7580(JDreamIII)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2011-57567 A (DAI ICHI KOGYO SEIYAKU CO., LTD.) 24 March 2011, claims, paragraphs [0001], [0021], [0032], [0034], [0035], [0043], [0045], [0048]-[0053], [0057] (Family: none) | 1-15<br>9 |
| A | 上谷幸治郎ら，液滴乾燥法によるナノセルロースの構造制御，セルロース学会第19回年次大会講演要旨集，01 July 2012, p. 108, (UETANI, Kojiro et al.), non-official translation (Structure control of nanocellulose by droplet drying method, Abstracts of the 19th Annual Meeting of the Cellulose Society of Japan) | 1-15 |
| X<br>Y | JP 2017-178888 A (KOSE CORPORATION) 05 October 2017, claims, paragraphs [0001], [0022]-[0025], [0029]-[0032] (Family: none) | 1-8, 10-15<br>9 |

☒ Further documents are listed in the continuation of Box C.          ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 30.09.2019 | 15.10.2019 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

| International application No. |
| --- |
| PCT/JP2019/035935 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2014-118521 A (KAO CORPORATION) 30 June 2014, claims, paragraphs [0001], [0035]-[0038], [0050] (Family: none) | 1-15 |
| A | JP 2005-53807 A (DAITO KASEI KOGYO KK) 03 March 2005, claims, paragraphs [0001], [0017]-[0041] (Family: none) | 1-15 |
| A | WO 2014/088072 A1 (NIPPON PAPER INDUSTRIES CO., LTD.) 12 June 2014, paragraphs [0001], [0011], [0018], [0032], [0025], [0026] & JP 2018-154837 A | 1-15 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016040264 A
- WO 2010092890 A
- WO 2017056908 A
- JP HEI9132601 A